# EUROPÄISCHE PATENTSCHRIFT

(11) **EP 0 587 534 B1**
(45) Veröffentlichungstag und Bekanntmachung des Hinweises auf die Patenterteilung: **23.10.1996**
(21) Anmeldenummer: 93810630.9
(22) Anmeldetag: 02.09.1993
(51) Int. Cl.: C07C 209/28

(54) **Verfahren zur Herstellung von Aminen**
Process for the preparation of amines
Procédé pour la préparation d'amines

(30) Priorität: 11.09.1992 CH 2866/92
(43) Veröffentlichungstag der Anmeldung: 16.03.1994
(73) Patentinhaber: CIBA-GEIGY AG, 4002 Basel (CH)
(72) Erfinder: Ouziel, Philippe, F-68130 Altkirch (FR)

(56) Entgegenhaltungen:
- GB-A- 1 055 616
- US-A- 2 366 534
- DATABASE WPI Week 8241, Derwent Publications Ltd., London, GB; AN 82-87393; & SU-A-887 563

## Beschreibung

Die vorliegende Erfindung betrifft ein Verfahren zur Herstellung von tertiären Aminen.

Es ist bereits z.B. aus der SU 887,563 bekannt, tertiäre Amine durch reduktive Aminierung von primären oder sekundären Aminen mit Formaldehyd und Ameisensäure herzustellen. Dabei wird das Amin zunächst mit Salzsäure vorbehandelt und anschliessend mit stöchiometrischen Mengen Ameisensäure und Formaldehyd reagieren gelassen; der pH-Wert des Reaktionsgemisches bleibt während der gesamten Umsetzung stark sauer. Man erhält das Produkt in einer Ausbeute von maximal 80 %, die verwendeten Temperaturen sind hoch und die Reaktionszeiten lang.

Es ist weiterhin bekannt, tertiäre Amine wie z.B. N-Methyl-N,N-diallylamin nach der sogenannten Eschweiler-Clarke-Reaktion herzustellen, indem man ein sekundäres Amin, einen Aldehyd und überschüssige Ameisensäure miteinander umsetzt. Die mit guter Ausbeute ablaufende Reaktion weist eine schlechte Oekobilanz, insbesondere hohe TOC-Werte, auf, da die Mutterlauge grosse Mengen von Ameisensäuresalzen enthält.

Es wurde nun überraschend gefunden, dass man die entsprechende Reaktion schnell, bei niedrigen Temperaturen und mit guter Ausbeute und Oekobilanz durchführen kann, wenn man die Reaktanden in etwa stöchiometrischen Mengen oder lediglich in geringem Ueberschuss einsetzt und den Reaktions-pH-Wert durch Zugabe einer Mineralsäure so steuert, dass das Reaktionsgemisch am Ende der Umsetzung einen neutralen bis leicht sauren Wert aufweist.

Gegenstand der vorliegenden Erfindung ist somit ein Verfahren zur Herstellung von Aminen der Formel 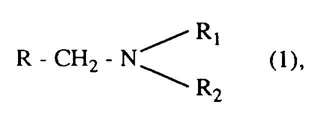 worin R Wasserstoff oder einen linearen oder verzweigten aliphatischen Rest bedeutet und R₁ und R₂ unabhängig voneinander je für einen gesättigten oder ungesättigten aliphatischen Rest stehen, dadurch gekennzeichnet, dass man ein 1 Moläquivalent Amin der Formel 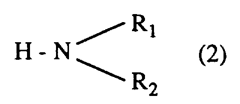 mit 0,8 bis 1,5 Moläquivalenten Aldehyd der Formel

R - CHO (3)

und 0,8 bis 2 Moläquivalente Ameisensäure in wässrigem Medium umsetzt und dabei den pH-Wert durch Zugabe einer Mineralsäure so einstellt, dass er am Ende der Umsetzung einen Wert von 3 bis 7 aufweist, und worin R, R₁ und R₂ in den Formeln (2) und (3) jeweils die oben angegebene Bedeutung haben.

Bei R als aliphatischem Rest handelt es sich z.B. um einen C₁-C₆-Alkylrest; Beispiele sind Methyl, Ethyl, n- oder iso-Propyl, n-, iso-, sec. oder tert.-Butyl oder geradkettiges oder verzweigtes Pentyl oder Hexyl. R steht als aliphatischer Rest bevorzugt für C₁-C₄-Alkyl und besonders bevorzugt für Methyl oder Ethyl.

R hat vorzugsweise die Bedeutung Methyl, Ethyl oder insbesondere Wasserstoff.

Stehen R₁ und/oder R₂ für einen gesättigten aliphatischen Rest, so kann dieser unabhängig die zuvor für R angegebenen Bedeutungen haben.

Bei R₁ und R₂ als ungesättigtem aliphatischem Rest handelt es sich unabhängig voneinander z.B. je um einen C₃-C₈-Alkenylrest. Beispiele sind Allyl, 1-Propenyl, iso-Propenyl, 2- oder 3-Methylallyl, 3-Butenyl oder 3,3-Dimetylallyl.

Die Reste R₁ und R₂ können verschieden oder, vorzugsweise, gleich sein und stehen besonders bevorzugt je für einen ungesättigten aliphatischen Rest; insbesondere bevorzugt ist für R₁ und R₂ je die Bedeutung Allyl.

Eine bevorzugte Ausführungsform der vorliegenden Erfindung betrifft die Herstellung von N-Methyl-N,N-diallylamin aus Diallylamin und Formaldehyd.

Die Wahl der Mineralsäure im vorliegenden Verfahren ist nicht kritisch. In Frage kommen alle üblichen Säure wie z.B. Schwefelsäure, Salzsäure, Salpetersäure oder Phosphorsäure. Bevorzugt ist die Verwendung von Salzsäure oder Schwefelsäure als Mineralsäure.

Man verwendet das Amin der Formel (2), den Aldehyd der Formel (3) und die Ameisensäure vorzugsweise in einem molaren Verhältnis von 1/1/1.

Das Verfahren wird vorteilhaft bei Normaldruck und einer erhöhten Temperatur, d.h. bei einer Temperatur von z.B. 40 bis 100°C und vorzugsweise von 60 bis 80°C, durchgeführt. Eine Variante des Verfahrens besteht darin, das Verfahren bei Ueberdruck, d.h. bei einem Druck von z.B. bis zu 5 atm., und Temperaturen über 100°C durchzuführen.

Man führt die Reaktion vorzugsweise so aus, dass der pH-Wert am Ende der Umsetzung einen Wert von 4 bis 6 aufweist. Die dafür benötigte Säuremenge beträgt ca. 0,3 bis 2,0 Mol-Aequivalente und vorzugsweise 0,5 bis 1 Mol-Aequivalent Mineralsäure pro Mol-Aequivalent Amin der Formel (2).

Eine besonders bevorzugte Ausführungsform der vorliegenden Erfindung betrifft ein Verfahren zur Herstellung von N-Methyl-N,N-diallylamin, welches dadurch gekennzeichnet ist, dass man ein 1 Moläquivalent N,N-Diallylamin mit 1 Moläquivalent (Para)formaldehyd und 1 Moläquivalent Ameisensäure in wässrigem Medium umsetzt und dabei den pH-Wert durch Zugabe von 0,5 bis 1 Moläquivalent Salzsäure oder Schwefelsäure so einstellt, dass er am Ende der Umsetzung einen Wert von 4 bis 6 aufweist.

Man geht erfindungsgemäss z.B. so vor, dass man den Aldehyd der Formel (3) und die Ameisensäure in Wasser vorlegt und die Reaktionslösung auf die gewünschte Reaktionstemperatur erhitzt. Dann wird das Amin der Formel (2) zugetropft; in der Folge steigt der pH-Wert des Reaktionsgemisches langsam an und wird bis zum Ende der Umsetzung mittels Zugabe von Mineralsäure auf einem Wert zwischen 3 und 7 und vorzugsweise zwischen 4 bis 6 gehalten. Es ist hierbei bevorzugt, mittels Zugabe des Amins einen bestimmten innerhalb des erfindungsgemässen Bereichs liegenden pH-Wert einzustellen und diesen Wert bis zum Ende der Reaktion durch Zugabe von Mineralsäure konstant zu halten.

Eine Variante des Verfahrens besteht darin, den Aldehyd der Formel (3), die Ameisensäure und Schwefelsäure (H₂SO₄) oder Phosphorsäure vorzulegen und das Amin der Formel (2) allmählich zuzutropfen; die Reaktion beginnt dann bei einem stark sauren pH-Wert, der im Lauf der Umsetzung langsam ansteigt und am Ende einen im erfindungsgemässen Bereich liegenden Wert aufweist.

Es ist weiterhin möglich, das Amin der Formel (2) und die Ameisensäure in Wasser vorzulegen und den Aldehyd der Formel (3) zuzutropfen. In diesem Fall kann die Mineralsäure zu Beginn oder während der Umsetzung zugegeben werden.

Die Reaktionslösung kann weitere Zusätze, z.B. übliche Antischaum- oder Netzmittel und Polymerisationsinhibitoren wie z.B. Hydrochinon-Derivate enthalten.

Die Reaktionszeiten variieren abhängig von den Reaktanden und den spezifischen Reaktionsbedingungen, betragen aber im allgemeinen 30 Minuten bis 5 Stunden und vorzugsweise 1 bis 3 Stunden.

Die Aufarbeitung der erhaltenen Reaktionsgemische geschieht in an sich bekannter Weise, z.B. indem man einen alkalischen pH-Wert einstellt, die sich bildenden zwei Phasen trennt und die organische das Amin enthaltende Phase einer Destillation unterwirft.

Die nach dem Verfahren erhältlichen Verbindungen stellen wertvolle Zwischenprodukte z.B. für die Synthese von Färbereihilfsprodukten dar.

Die folgenden Beispiele verdeutlichen die vorliegende Erfindung. Teile bedeuten Gewichtsteile, Prozente Gewichtsprozente.

Beispiel 1: In einem geeigneten Reaktionsgefäss, welches mit Rührer, Thermometer, Rückflusskühler, eine pH-Elektrode und einem Eingang für die Salzsäure-Zugabe ausgestattet ist, werden 219,4 Teile 84%ige Ameisensäure, 125,3 Teile 95,5%iger Paraformaldehyd, 249 Teile Wasser, 0,2 Teile Hydrochinon und 2 Teile eines Antischaummittels (2,4,7,9-Tetramethyl-5-decyn-4,7-diol) vorgelegt und die erhaltene Suspension auf 65°C erhitzt. Man tropft innerhalb von ca. 45 min. 388,8 Teile Diallylamin bei einer Temperatur von ca. 58-62°C zu; dabei steigt der pH-Wert langsam an und wird, nachdem er einen Wert von 5,5 erreicht hat, durch Zugabe von 37%iger Salzsäure konstant bei diesem Wert gehalten. Nach beendeter Diallylamin-Zugabe wird das Reaktionsgemisch auf 65-70°C erhitzt und ca. 2 Stunden bei dieser Temperatur und pH 5,5 rühren gelassen.

Man lässt auf Raumtemperatur abkühlen und stellt durch Zugabe von Natriumhydroxidlösung einen pH-Wert ≥ 12 ein; es bilden sich zwei Phasen, die getrennt werden. Die organische Phase wird mit 0,1 Teilen Hydrochinon stabilisiert, das darin enthaltene Wasser azeotrop abdestilliert und anschliessend das N-Methyl-N,N-diallylamin destillien. Siedepunkt 112-115°C; Ausbeute 406 Teile (91,4%)Produkt mit einer Reinheit von 99,8%. Der TOC-Wert der Mutterlauge liegt bei ≤ 8000 ppm.

Beispiel 2: In der im Beispiel 1 beschriebenen Apparatur werden 215,3 Teile 85%ige Ameisensäure, 125,5 Teile 95,5%iger Paraformaldehyd, 290 Teile Wasser, 204,3 Teile 96%ige H₂SO₄ und 0,2 Teile Hydrochinon vorgelegt und die erhaltene Suspension auf 75°C erhitzt. Man tropft 388,8 Teile Diallylamin innerhalb von ca. 30 min. zu und hält dabei die Temperatur bei 70 bis 75°C. Nach Beendigung der Diallylamin-Zugabe lässt man noch 3 Stunden bei 75°C nachrühren und isoliert dann das Produkt wie im Beispiel 1 beschrieben. Ausbeute 408 Teile (91,9 %) N-Methyl-N,N-diallylamin mit einer Reinheit von 99,7 %; TOC-Wert der Mutterlauge ≤ 9800 ppm.

Vergleichsbeispiel: Ein Versuch wird nach Eschweiler-Clarke durchgeführt; die Reaktionsbedingungen und Ergebnisse sind in der Tabelle aufgeführt und denen der Beispiele 1 und 2 gegenübergestellt. Die Tabelle enthält ausserdem die Angaben aus der SU 887,563, Beispiel 4.

| Verfahren | Eschweiler Clarke | SU 887,563 Beispiel 4 | Beispiel 1 | Beispiel 2 |
|---|---|---|---|---|
| Diallylamin (Mol) | 1 | 1 | 1 | 1 |
| (Para)formaldehyd(Mol) | 1,2 | 1 | 1 | 1 |
| Ameisensäure (Mol) | 2,4 | 1 | 1 | 1 |
| HCl bzw. H₂SO₄ (Mol) | --- | 1 | 0,9 | 0,5 |
| Reaktionstemp. (°C) | 80 | 100 | 65 | 75 |
| Reaktionszeit (Std.) | 3 | 9 | 2 | 3 |
| End-pH-Wert | 3,9 | n.a.¹⁾ | 5,5 | 4,5 |
| TOC-Wert der Mutterlauge (ppm) | ≥60000 | n.a. | 8000 | 9800 |
| Ausbeute (% der Th.) | 90-92 | 78 | 91,4 | 91,9 |

| | | | | |
|---|---|---|---|---|
| ¹⁾gemäss eigener Nacharbeitung wesentlich geringer als 3. | | | | |

## Patentansprüche

1. Verfahren zur Herstellung von Aminen der Formel 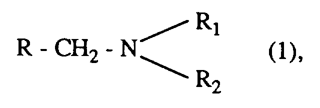 worin R Wasserstoff oder einen linearen oder verzweigten aliphatischen Rest bedeutet und R₁ und R₂ unabhängig voneinander je für einen gesättigten oder ungesättigten aliphatischen Rest stehen, dadurch gekennzeichnet, dass man ein 1 Moläquivalent Amin der Formel 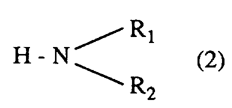 mit 0,8 bis 1,5 Moläquivalenten Aldehyd der Formel
R - CHO (3)
und 0,8 bis 2 Moläquivalenten Ameisensäure in wässrigem Medium umsetzt und dabei den pH-Wert durch Zugabe einer Mineralsäure so einstellt, dass er am Ende der Umsetzung einen Wert von 3 bis 7 aufweist, und worin R, R₁ und R₂ in den Formeln (2) und (3) jeweils die oben angegebene Bedeutung haben.

2. Verfahren gemäss Anspruch 1, dadurch gekennzeichnet, dass R Wasserstoff, Methyl oder Ethyl bedeutet.

3. Verfahren gemäss Anspruch 1 oder 2, dadurch gekennzeichnet, dass R₁ und R₂ unabhängig voneinander jeweils Allyl, 1-Propenyl, iso-Propenyl, 2- oder 3-Methallyl, 3-Butenyl oder 3,3-Dimethylallyl bedeuten.

4. Verfahren gemäss einem der Ansprüche 1 bis 3, dadurch gekennzeichnet, dass R Wasserstoff und R₁ und R₂ jeweils Allyl bedeuten.

5. Verfahren gemäss einem der Ansprüche 1 bis 4, dadurch gekennzeichnet, dass man Salzsäure oder Schwefelsäure als Mineralsäure verwendet.

6. Verfahren gemäss einem der Ansprüche 1 bis 5, dadurch gekennzeichnet, dass man das Amin der Formel (2), den Aldehyd der Formel (3) und die Ameisensäure im molaren Verhältnis von 1/1/1 verwendet.

7. Verfahren gemäss einem der Ansprüche 1 bis 6, dadurch gekennzeichnet, dass man die Umsetzung bei einer Temperatur von 40 bis 100°C, vorzugsweise 60 bis 80°C, ausführt.

8. Verfahren gemäss einem der Ansprüche 1 bis 7, dadurch gekennzeichnet, dass der pH-Wert am Ende der Umsetzung 4 bis 6 beträgt.

9. Verfahren gemäss einem der Ansprüche 1 bis 8, dadurch gekennzeichnet, dass man 0,3 bis 2,0, vorzugsweise 0,5 bis 1, Moläquivalente Mineralsäure pro Moläquivalent Amin der Formel (2) einsetzt.

10. Verfahren gemäss Anspruch 1 zur Herstellung von N-Methyl-N,N-diallylamin, dadurch gekennzeichnet, dass man ein 1 Moläquivalent N,N-Diallylamin mit 1 Moläquivalent (Para)formaldehyd und 1 Moläquivalent Ameisensäure in wässrigem Medium umsetzt und dabei den pH-Wert durch Zugabe von 0,5 bis 1 Moläquivalent Salzsäure oder Schwefelsäure so einstellt, dass er am Ende der Umsetzung einen Wert von 4 bis 6 aufweist.

## Claims

1. A process for the preparation of an amine of formula 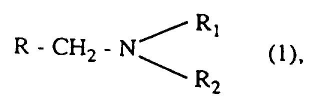 wherein R is hydrogen or a linear or branched aliphatic radical, and R₁ and R₂ are each independently of the other a saturated or unsaturated aliphatic radical, which comprises reacting 1 molar equivalent of an amine of formula 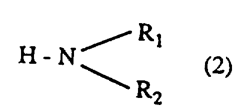 with 0.8 to 1.5 molar equivalents of an aldehyde of formula
R-CHO (3)
and 0.8 to 2 molar equivalents of formic acid, in an aqueous medium, and adjusting the pH by addition of a mineral acid such that, at the conclusion of the reaction, the pH is in the range from 3 to 7, and wherein R, R₁ and R₂ in formulae (2) and (3) are each as defined above.

2. A process according to claim 1, wherein R is hydrogen, methyl or ethyl.

3. A process according to claim 1 or 2, wherein R₁ and R₂ are each independently of the other allyl, 1-propenyl, isopropenyl, 2- or 3-methallyl, 3-butenyl or 3,3-dimethylallyl.

4. A process according to any one of claims 1 to 3, wherein R is hydrogen and R₁ and R₂ are each allyl.

5. A process according to any one of claims 1 to 4, wherein the mineral acid is hydrochloric acid or sulfuric acid.

6. A process according to any one of claims 1 to 5, wherein the amine of formula (2), the aldehyde of formula (3) and the formic acid are used in the molar ratio of 1/1/1.

7. A process according to any one of claims 1 to 6, wherein the reaction is carried out in the temperature range from 40 to 100°C, preferably from 60 to 80°C.

8. A process according to any one of claims 1 to 7, wherein at the conclusion of the reaction the pH is in the range from 4 to 6.

9. A process according to any one of claims 1 to 8, wherein 0.3 to 2.0 molar equivalents, preferably 0.5 to 1 molar equivalent, of mineral acid is used per molar equivalent of amine of formula (2).

10. A process according to claim 1 for the preparation of N-methyl-N,N-diallylamine, which comprises reacting 1 molar equivalent of N,N-diallylamine with 1 molar equivalent of (para)formaldehyde and 1 molar equivalent of formic acid, in aqueous medium, and adjusting the pH by addition of 0.5 to 1 molar equivalent of hydrochloric acid or sulfuric acid such that, at the conclusion of the reaction, the pH is in the range from 4 to 6.

## Revendications

1. Procédé pour la préparation d'amines de formule 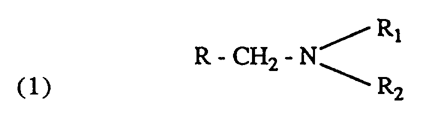 dans laquelle R représente un atome d'hydrogène ou un résidu aliphatique linéaire ou ramifié et R₁ et R₂ représentent indépendamment l'un de l'autre un résidu aliphatique saturé ou insaturé, caractérisé en ce que l'on fait réagir 1 équivalent molaire d'une amine de formule 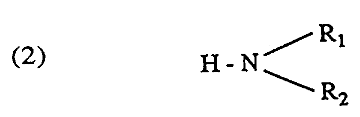 avec 0,8 à 1,5 équivalent molaire d'un aldéhyde de formule
(3) R-CHO
et avec 0,8 à 2 équivalents molaires d'acide formique dans un milieu aqueux et en ce que l'on ajuste le pH par addition d'un acide minéral de manière à ce que, à la fin de la réaction, il soit compris entre 3 et 7, et dans laquelle R, R₁ et R₂, dans les formules (2) et (3), ont la signification indiquée ci-dessus.

2. Procédé conforme à la revendication 1, caractérisé en ce que R représente un atome d'hydrogène, un groupe méthyle ou éthyle.

3. Procédé conforme à la revendication 1 ou 2, caractérisé en ce que R₁ et R₂ représentent indépendamment l'un de l'autre chacun un groupe allyle, 1-propényle, iso-propényle, 2- ou 3-méthylallyle, 3-butényle ou 3,3-diméthylallyle.

4. Procédé conforme à une des revendications 1 à 3, caractérisé en ce que R représente un atome d'hydrogène et R₁ et R₂ chacun un groupe allyle.

5. Procédé conforme à une des revendications 1 à 4, caractérisé en ce que l'on utilise comme acide minéral de l'acide chlorhydrique ou de l'acide sulfurique.

6. Procédé conforme à une des revendications 1 à 5, caractérisé en ce que l'on utilise l'amine de formule (2), l'aldéhyde de formule (3) et l'acide formique en un rapport molaire de 1/1/1.

7. Procédé conforme à une des revendications 1 à 6, caractérisé en ce que l'on réalise la réaction à une température comprise entre 40 et 100 °C, de préférence entre 60 et 80 °C.

8. Procédé conforme à une des revendications 1 à 7, caractérisé en ce que le pH, à la fin de la réaction, est compris entre 4 et 6.

9. Procédé conforme à une des revendications 1 à 8, caractérisé en ce que l'on utilise de 0,3 à 2,0, de préférence de 0,5 à 1 équivalent molaire par équivalent molaire de l'amine de formule (2).

10. Procédé conforme à la revendication 1 pour la préparation de la N-méthyl-N,N-diallylamine, caractérisé en ce que l'on fait réagir 1 équivalent molaire de N,N-diallylamine avec 1 équivalent molaire de (para)formaldéhyde et 1 équivalent molaire d'acide formique dans un milieu aqueux tout en ajustant le pH par addition de 0,5 à 1 équivalent molaire d'acide chlorhydrique ou d'acide sulfurique de manière à ce que, à la fin de la réaction, il soit compris entre 4 et 6.
